# EUROPEAN PATENT APPLICATION

(11) **EP 0 745 368 A1**
(43) Date of publication of application: **04.12.1996**
(21) Application number: 95108431.8
(22) Date of filing: 01.06.1995
(51) Int. Cl.: A61F 13/58, A61F 13/56

(54) **Disposable absorbent article having a shaped panty-fastening adhesive**

(71) Applicant: THE PROCTER & GAMBLE COMPANY, Cincinnati, Ohio 45202 (US)
(72) Inventor: Hagemeister, Eric Peter, D-74532 Ilshofen (DE); Lender, Horst Andreas, D-91740 Röckingen (DE); Hundorf, Harald Hermann, D-61352 Bad Homburg (DE); Plumley, Julian Ashton, D-74589 Satteldorf (DE)
(74) Representative: Hirsch, Uwe Thomas M.H.

(57) **Abstract**

The present invention relates to machine made disposable absorbent articles having a panty-fastening adhesive on their garment-facing surface. The panty-fastening adhesive has a shape which is at least partially non-linear. This new shape can be provided by a novel adhesive printing process for disposable absorbent articles.

## Description

### Field of the invention

The present invention relates to machine made disposable absorbent articles such as sanitary napkins, panty liners, catamenials incontinence inserts, and diaper inserts. In particular the present invention relates to disposable absorbent articles having a panty-fastening adhesive on their garment-facing surface. The panty-fastening adhesive has an at least partially non-linear shape. This new shape can be provided by a novel adhesive printing process disclosed in parallel patent applications entitled "Adhesive printing for disposable absorbent articles" or "Continuous adhesive printing onto discontinuous series of disposable absorbent articles".

### Background of the invention

Absorbent articles such as sanitary napkins, panty liners, catamenials, incontinence inserts and diaper inserts are commonly provided with an adhesive on their garment-facing surface to attach them during their usage period to a garment of the user. In particular sanitary napkins and panty liners are commonly provided with a pressure sensitive, thermoplastic, adhesive which attaches to the undergarment of the wearer and thereby improves fit and comfort of the product for the wearer.

Typically these products are made by high speed machinery. The machinery includes equipment which adds the adhesive in a very fast and efficient manner, ensuring consistency of the absorbent products over large quantities thereof.

A common way of providing an adhesive is a slot-coating or spraying of the adhesive onto a continuously conveyed thread of material. The surface of this material, coated with adhesive, later provides the garment-facing surface for the disposable absorbent articles. Necessarily this adhesive application creates adhesive areas with linear side edges which are parallel to the machine direction axis of the product.

In order to save adhesive material and to provide adhesive free areas for easier handling a discontinuous adhesive coating by sequentially turning on and off the adhesive supply equipment are known. This, however, still is limited to provide a panty-fastening adhesive area of linear side edges parallel to the machine direction axis.

The applicants have now found a way of providing panty-fastening adhesive areas to machine made disposable absorbent articles which areas are not limited in their shape by the equipment and process considerations of the past.

The problem of shaped panty-fastening adhesive areas has previously been considered in the art but always accepted linear side edges due to process restrictions. Liberty in the shape of panty-fastening adhesive areas was only achieved for products in which the periphery of the product and the periphery of the panty-fastening adhesive were identical. These products were made by coating the adhesive somewhat wider than the outermost edged of the final product periphery relative to the machine direction axis and cutting the product together with the panty-fastening adhesive into the desired shape. Naturally, considerations for the product shape were dominant in these designs rather than consideration for the shape of panty-fastening adhesive areas.

It is therefore an objective of the present invention to provide disposable absorbent articles having a panty-fastening adhesive on their garment-facing surface which panty-fastening adhesive can be provided in any desired shape. In particular, panty-fastening adhesives comprising non-linear peripheral edges which are within the peripheral edge of the disposable absorbent article are achieved for the first time by the present invention.

### Summary of the invention

The present invention provides machine made disposable absorbent articles which have been made in a machine direction and hence have a machine direction axis. The articles comprise a body-facing surface and a garment-facing surface on which an adhesive area is provided for attaching the article to an underlying garment such as an undergarment.

The garment-facing surface has an endless peripheral edge which typically also defines the periphery of the absorbent article. The adhesive area also has an endless peripheral edge. The peripheral edge of the adhesive area comprises inner parts which are not coinciding with the peripheral edge of the garment-facing surface. These inner parts of the adhesive area comprise at least one non-linear portion. Preferably the inner parts are nowhere parallel to the machine direction axis. "Nowhere parallel" naturally includes inner parts having turning points which are only tangentially parallel to the machine direction. Preferably the peripheral edge of the adhesive area does not coincide anywhere with the peripheral edge of the underlying garment-facing surface.

The adhesive area beneficially can also form an area which encloses at least one smaller area which is at least partially free of adhesive. Also the absorbent article can comprise a multitude of separated adhesive areas, in particular when the garment-facing surface of the article and the whole article are made to be breathable. For these adhesive areas the outer periphery or the inner periphery can comprise the non-linear portions.

Especially desirable shapes of the adhesive area are indicated in the attached drawings and include such shapes as irregular spots, polygonal dots, square dots, rectangular dots, circular dots, oval dots, circular rings, oval rings or any combination thereof.

The adhesive area on the disposable absorbent article is typically covered by a releasable protective cover mean such as a siliconised release paper prior to use of the article. For disposable absorbent articles all other common features are also desirable in the context of the present invention. The shaped panty-fastening adhesive according to the present invention is particularly desirable in combination with sanitary napkins or panty liners having wings or side wrapping elements.

### Brief description of the drawings

Figure 1 shows the garment-facing surface of a shaped sanitary napkin having a shaped panty-fastening adhesive area with a reduced adhesive area all around.

Figure 2 shows an alternative embodiment of a panty-fastening adhesive area configuration which includes a multitude of circular dots.

Figure 3 shows the garment-facing surface of a sanitary napkin having wings with a panty-fastening adhesive area reaching into the wings while having a reduced central adhesive area.

Figures 4 shows the garment-facing surface of a sanitary napkin according to the prior art having only linear side edges parallel to the machine direction axis.

### Detailed description of the invention

The present invention relates to absorbent articles which in use are joined to a garment by an adhesive area on the garment facing surface of the absorbent article. Based on the adhesive printing technique disclosed in copending applications entitled "Adhesive printing for disposable absorbent articles" or Continuous adhesive printing onto discontinuous series of disposable absorbent articles" it has become possible to create panty-fastening adhesive shapes which comprise a non-linear part in mass production. In particular this allows for the first time to provide machine mad absorbent articles with any desirable shapes of the panty-fastening adhesive on their garment-facing surface.

"Mass production" or "machine made" as used herein refers to a high speed making process when producing absorbent articles. Typically the machines transport a continuous thread of materials (representing the absorbent articles prior to severing them from the continuous thread) at a speed of at least 0.5 m/s, preferably at least 1.5 m/s. For articles of 300 mm length (including trim) this results in at least 100, preferably at least 300 articles per minute.

The absorbent article has a body facing surface, typically provided by a liquid permeable substrate of fibrous or film like structure; a garment facing surface, preferably provided by a liquid impermeable, but breathable substrate and an absorbent structure placed between the body facing surface and the garment facing surface. In the figures the absorbent article has a longitudinal axis (10) and a lateral axis (11) and can comprise any of the components or features usual in the art. In particular side wrapping elements, side flap components, or wings as well as any sort of extensibility or elastication feature can be comprised in the absorbent articles of the present invention.

The disposable article for absorbent liquid is described below by reference to a sanitary napkin or panty liner. However products such as adult or baby diaper inserts comprising fastening adhesives can similarly benefit from the process of the present invention.

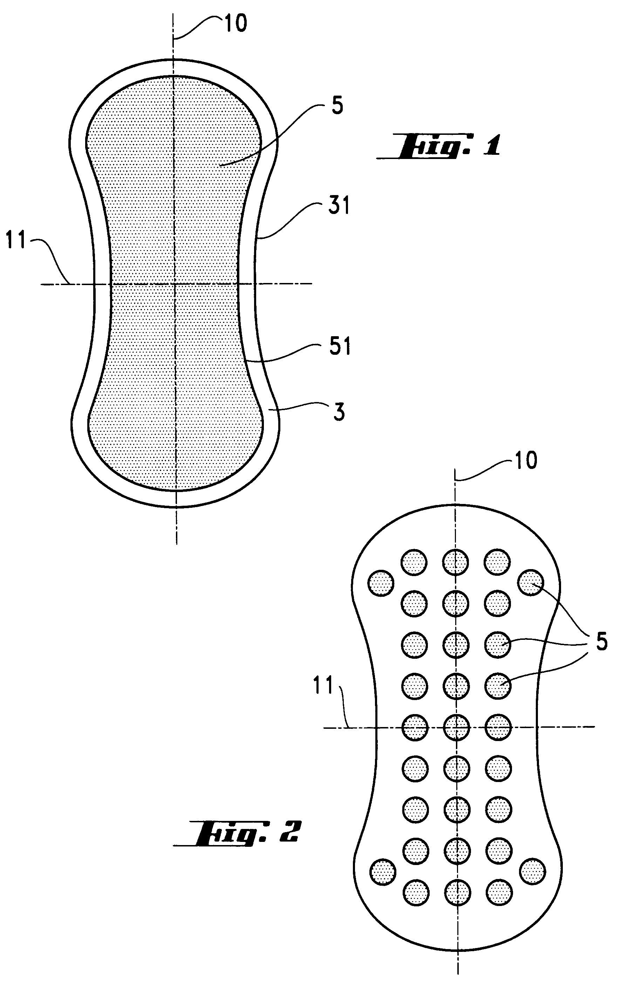

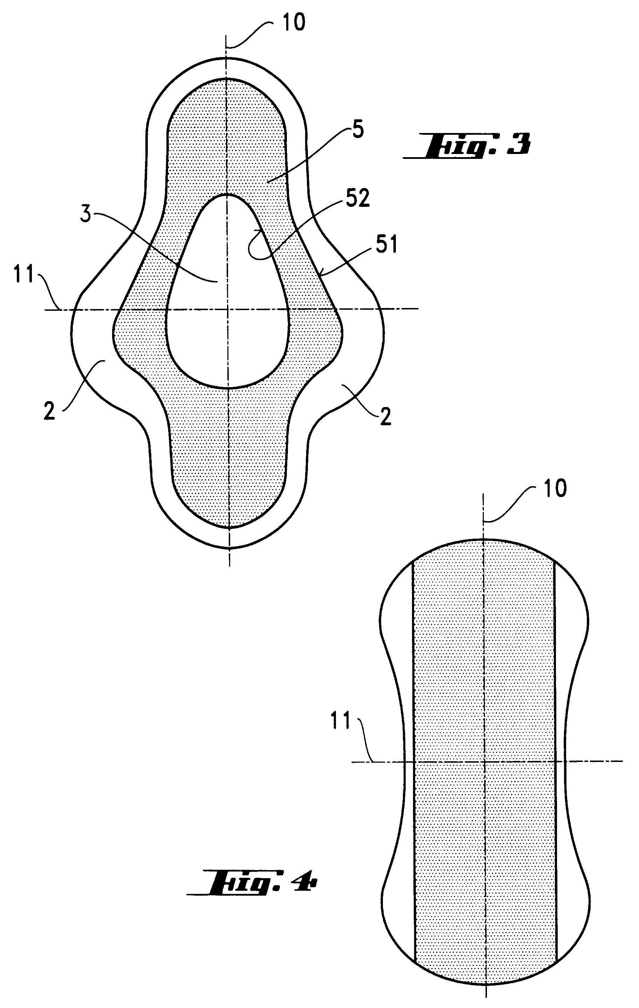

A preferred sanitary napkin or panty liner made according to the present invention has a pair of side wrapping elements (or "undergarment covering components") designated (2) in figure 3. They provide coverage of the wearer's panties to reduce side soiling (i.e., staining of the edges of the panty crotch) and are typically smaller than conventional flaps or wings.

The function of side wrapping elements, whether integral or joined to the article after being formed separately, is further improved by rendering them extensible in one or both directions parallel to the longitudinal axis (10) and/or lateral axis (11). The extensibility can be provided across all or only part of the side wrapping elements and can be achieved by pleating or ring-rolling those parts which are to be rendered extensible.

A typical sanitary napkin or panty liner comprises a main body portion comprising a liquid pervious topsheet, a liquid impervious backsheet joined to the topsheet, and an absorbent core positioned between the topsheet and the backsheet. On the backsheet is provided an adhesive area providing the panty-fastening adhesive.

### Topsheet

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps, side wrapping elements or wings.

When referring to the topsheet a multi layer structure or a mono layer structure is contemplated. The hybrid topsheet mentioned above is such a multi layer design but other multi layer topsheets such as primary and secondary topsheet designs are also considered.

### Absorbent structure

The absorbent structure can include the following components: (a) optionally a primary fluid distribution layer preferably together with a secondary optional fluid distribution layer; (b) a fluid storage layer; (c) optionally a fibrous ("dusting") layer underlying the storage layer; and (d) other optional components.

### a Primary/Secondary Fluid Distribution Layer

One optional component of the absorbent structure according to the present invention is a primary fluid distribution layer and a secondary fluid distribution layer. The primary distribution layer typically underies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared form polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins/panty liners.

An embodiment of the absorbent structure made according to the present invention comprises a double layer tissue laminate formed by folding the tissue onto itself. These layers can be joined to each other. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent structure according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent structure. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent structure according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

### Backsheet

The backsheet (3) primarily prevents the exudates absorbed and contained in the absorbent structure from wetting articles that contact the absorbent product such as underpants, pants, pyjamas and undergarments. The backsheet (3) is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings as shown in Figure 3.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further,. the backsheet can permit vapours to escape from the absorbent structure, i.e. be breathable, while still preventing exudates from passing through the backsheet. Also breathable backsheets comprising several layers, e.g. film plus non-woven structures, can be used and are preferably joined to each other.

### The panty-fastening-adhesive

The backsheet typically forms the garment facing surface of the absorbent article on which the panty fastening adhesive is placed. Panty-fastening-adhesives can comprise any adhesive or glue used in the art for such purposes with pressure-sensitive adhesives being preferred.

It is important that the adhesive is suitable for printing onto the garment facing surface of the absorbent article if the adhesive is to be applied by adhesive printing disclosed in copending application entitled "Adhesive printing for disposable absorbent articles" or "Continuous adhesive printing onto discontinuous series of disposable absorbent articles".

Suitable adhesives are Savare LA203 and LA303 of the Savare I.C. company of Milan in Italy, Coramelt 867 by Koemmerling in Pirmasens in Germany and Fuller H-2238ZP manufactured by the H.B. Fuller Co of Lueneburg in Germany. Suitable adhesive fasteners are also described in U.S. Patent 4,917,697.

It is a key feature of the present invention that the shape of the panty-fastening adhesive area, designated (5) in the figures, comprises a non-linear portion which is not coincident with the periphery of the garment facing surface of the absorbent article. As can be seen from figure 1, the panty-fastening adhesive is not coextensive with the garment facing surface of the sanitary napkin. In particular, the design of figure 1 leaves an adhesive-free border all around between the periphery (51) of the panty-fastening adhesive area (5) and the periphery (31) of the garment facing surface (3).

With the development of adhesive printing it has become possible to provide such panty-fastening adhesive shapes to machine made articles. Previously it was usual to provide a patch of adhesive onto the garment facing surface of an absorbent article by spraying or coating continuously or even discontinuously adhesive onto a continuous running thread of sanitary napkins not yet severed from each other.

This necessarily produced linear side edges of the panty-fastening adhesive as shown in prior art figure 4. The periphery of the panty-fastening adhesive (provided it did not extend to the periphery of the garment facing surface) comprised only linear portions, either parallel to the machine direction axis of the sanitary napkin or perpendicular to this axis.

The present development for the first time provides non-linear portions of the panty-fastening adhesive area to machine made sanitary napkins and other absorbent articles. This allows full liberty for designing the shape of the panty fastening adhesive.

Once the liberty for deciding the shape of the panty-fastening adhesive is achieved, it is possible to optimise the panty-fastening adhesive shape of the particular desired purposes and functionality of the absorbent article.

For example sanitary napkins typically worn in tight fitting underwear can be optimised by only requiring a rim around the periphery of the garment facing surface with panty-fastening adhesive. In the other hand, sanitary napkins designed for loose fitting undergarments could be provided with a pattern of adhesive dots thereby reducing adhesive quantity and allowing for elasticated garment facing surfaces due to the non-adhered parts between the dots. It is also possible that shapes indicating front or rear of the article or providing usage instruction indications can be provided.

If the backsheet provides the sanitary napkin with breathability it is important that the panty fastening adhesive does not close or cog the whole garment facing side of the backsheet. For this purpose it is possible to use an adhesive in such a pattern to only seal off a fraction of the garment facing surface by adhesive. On the other hand the minimum adhesive coverage of the garment facing surface to provide the benefit of proper attachment to the undergarment of an absorbent article can easily be defined by those skilled in the art.

Therefore, preferably the panty fastening adhesive (5) is applied in intermittent patterns such as for example intermittent dots as shown in figure 2, intermittent strips, or other designed shapes like circles. This permits the sanitary napkin to remain breathable and ensures an optimal fiction of the sanitary napkin to the undergarment. If the adhesive encircles an adhesive free area than an inner periphery (52) exists which can comprise the non-linear portion of the panty fastening adhesive.

If protective side flaps are present then they can have optional fasteners thereon for additional security. The optional protective side flap fasteners can form an integral area with the main panty fastening adhesive as shown in figure 3 or form separate patterns. According to the present invention the adhesive can be identical or different depending on the desired adhesive connection. The fasteners assist the protective side flaps to stay in position after they wrap around the edges of the crotch surface of the undergarment.

Prior to use of the absorbent article the panty fastening adhesive is typically protected from contamination and from sticking to any surface where this is not desired by a protective cover means such as a silicone coated release paper, a plastic film or any other easily removable cover. The protective cover means can be provided as a single piece or in a multitude of pieces e.g. to cover the individual adhesive areas. It also can perform other functions such as provide individualised packaging for the article or provide a disposal function.

## Claims

1. Machine made disposable absorbent article comprising a body facing surface and a garment facing surface, said article comprising a machine direction axis, said article comprising an adhesive area on said garment facing surface,
said garment facing surface comprising an endless peripheral edge and said adhesive area comprising an endless peripheral edge, said peripheral edge of said adhesive area comprising inner parts which are not coincident with said peripheral edge of said garment facing surface,
and said article being characterised in that
at least one of said inner parts comprises at least one non-linear portion.

2. A disposable absorbent article according to claim 1 characterised in that said inner parts are nowhere parallel to said machine direction axis of said article.

3. A disposable absorbent article according to any of the preceding claims characterised in that said article comprises a releasable protective cover means overlaying said adhesive area.

4. A disposable absorbent article according to any of the preceding claims characterised in that said article is a sanitary napkin or a pantyliner.

5. A disposable absorbent article according to claim 4 characterised in that said article has wings and said adhesive area either extends to said wings or said wings are provided with a separate adhesive area.

6. A disposable absorbent article according to claim 5 characterised in that said adhesive area extending to said wings comprises said non-linear potions.

7. A disposable absorbent article according to any of the preceding claims characterised in that said peripheral edge of said adhesive area nowhere coincides with said peripheral edge of said garment facing surface.

8. A disposable absorbent article according to any of the preceding claims characterised in that said adhesive area encircles at least one area which is at least partially free of adhesive.

9. A disposable absorbent article according to any of the preceding claims characterised in that said article comprises a multitude of separated adhesive areas, preferably said disposable absorbent article being breathable.

10. A disposable absorbent article according to claim 9 characterised in that said adhesive areas are selected from the shapes of irregular spots, polygonal dots, square dots, rectangular dots, circular dots, oval dots, circular rings, oval rings or any combination thereof.
